(19) Europäisches Patentamt
European Patent Office
Office européen des brevets

(11) Veröffentlichungsnummer: **0 175 289**
**A2**

# (12) EUROPÄISCHE PATENTANMELDUNG

(21) Anmeldenummer: **85111556.8**

(22) Anmeldetag: **12.09.85**

(51) Int. Cl.[4]: **A 61 K 45/02**

(30) Priorität: **19.09.84 DE 3434345**

(43) Veröffentlichungstag der Anmeldung: **26.03.86 Patentblatt 86/13**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Seeger, Karl, Dr., Schwalbenweg 9, D-6238 Hofheim am Taunus (DE)**
Erfinder: **Fülberth, Werner, Dr., Theodor-Storm-Strasse 11, D-6233 Kelkheim (Taunus) (DE)**
Erfinder: **Leineweber, Michael, Dr., Heimchenweg 74, D-6230 Frankfurt am Main 80 (DE)**
Erfinder: **Obermeier, Rainer, Dr., langenhainer Weg 14, D-6234 Hattersheim am Main (DE)**
Erfinder: **Rolly, Heinrich, Dr., Am Tulpengarten 18, D-6233 Kelkheim (Taunus) (DE)**

(54) **Bekämpfung von Durchfällen mit Alpha-Interferonen.**

(57) α-Interferone sind wirksam gegen Durchfallerkrankungen. Das Interferon wird zweckmässig oral in einer magensaftresistenten Zubereitung verabreicht.

EP 0 175 289 A2

Bekämpfung von Durchfällen mit α-Interferonen

Es ist bekannt, daß die Interferone gegen Viren wirksam sind und das Immunsystem stimulieren können. Es ist weiterhin bekannt, daß das menschliche Leukozyten-Interferon bzw. α-Interferon, wie es durch Induktion menschlicher Leukozytenzellen gebildet wird, keine einheitliche Verbindung darstellt, sondern aus mehreren interferonaktiven Komponenten besteht. Es wurden auch schon künstliche "Hybride" mit Anteilen von α-Interferonen hergestellt. So beschreibt beispielsweise die veröffentlichte Europäische Patentanmeldung 51 873 solche Hybride und ihre gentechnologische Herstellung.

Es ist bekannt, daß Interferone ganz allgemein gegen Viren wirksam sind, daß die Wirkung aber in vielen Fällen wirtsspezifisch ist, wobei also ein Human-Interferon nicht notwendigerweise die gleiche Wirkung in anderen Säugetieren zeigt. Andererseits wird den Hybriden ein breites Wirtsspektrum zugeschrieben.

Es wurde nun gefunden, daß α-Interferone gegen Durchfallerkrankungen wirksam sind, insbesondere zur Behandlung von Durchfällen, die durch Viren verursacht oder mitverursacht werden. Die Durchfälle hören in kurzer Zeit auf und in den Exkrementen sind keine Viren mehr nachweisbar.

Vorteilhaft wird das α-Interferon in einer oralen Zubereitung verabreicht, welche gewährleistet, daß das Interferon, das ja ein Polypeptid darstellt, nicht im Magen abgebaut wird. Zweckmäßig sind magensaft-resistente Kapseln, die im Darmmilieu aufgelöst werden und die Wirksubstanz freisetzen. Möglich sind weiterhin geeignete Puffersysteme.

Die Dosis ist abhängig von der Schwere der Erkrankung, dem Alter und Gewicht des Patienten und liegt zweckmäßig im Bereich von $10^5$ bis $10^8$ IFE pro Tag. Es kann beispielsweise bei einem Erwachsenen drei Tage lang täglich eine Kapsel mit 0,1 bis 1 x $10^8$ IFE verabreicht werden.

Bei der Anwendung der Erfindung auf andere Säugetiere bzw. Warmblüter ist zu prüfen, ob ein Human-Interferon, vorzugsweise ein Hybrid-α-Interferon, wirksam ist. Diese Produkte können in bekannter Weise gentechnologisch hergestellt werden (Europäische Patentanmeldung mit der Veröffentlichungsnummer 51873). Andernfalls kann von der Erfindung auch mit einem artspezifischen α-Interferon Gebrauch gemacht werden.

Bevorzugte α-Interferone entsprechen der Formel

$$H_2N\text{-}Met\text{-}Cys^1\text{-}X\text{-}P\text{-}Y\text{-}B\text{-}Z\text{-}COOH,$$

in der X im wesentlichen den Aminosäuren 2 bis 46 des menschlichen Lymphoblasten-Interferon (LbIF) entspricht, Y im wesentlichen den Aminosäuren 60 bis 85 von LbIF entspricht, Z im wesentlichen den Aminosäuren 112 bis 166 von LbIF entspricht, P im wesentlichen den Aminosäuren 47 bis 59 eines menschlichen αC-, αF- oder αI-Typs des Leukozyten-Interferons (LeIF) entspricht und B im wesentlichen den Aminosäuren 86 bis 111 von LeIF entspricht. Die Formulierung "im wesentlichen entspricht" besagt, daß Variationen, die die biologische Aktivität nicht oder nicht wesentlich beeinträchtigen, zulässig sind, also ein Austausch, Weglassen oder Hinzufügen von Aminosäuren, wie es bei der gentechnologischen Synthese von Polypeptiden an sich bekannt ist.

B ist insbesondere $Ser^{86}$-Thr-Glu-Leu-$Tyr^{90}$-Gln-Gln-Leu-Asn-$Asp^{95}$-Leu-Glu-Ala-Cys-$Val^{100}$-Ile-Gln-Glu-Val-$Gly^{105}$-Val-Glu-Glu-Thr-$Pro^{110}$-Leu oder $Ser^{86}$-Thr-Glu-Leu-$Tyr^{90}$-Gln-

Gln-Leu-Asn-Asn$^{95}$-Leu-Glu-Ala-Cys-Val$^{100}$-Ile-Gln-Glu-Val-Gly$^{105}$-Met-Glu-Glu-Thr-Pro$^{110}$-Leu, P ist insbesondere Gln$^{47}$-Phe-Gln-Lys$^{50}$-Ala-Gln-Ala-Ile-Ser$^{55}$-Val-Leu-His-Glu$^{59}$, oder Gln$^{47}$-Phe-Gln-Lys$^{50}$-Thr-Gln-Ala-Ile-Ser$^{55}$-Val-Leu-His-, X ist insbesondere Asp$^{2}$-Leu-Pro-Gln$^{5}$-Thr-His-Ser-Leu-Gly$^{10}$-Asn-Arg-Arg-Ala-Leu$^{15}$Ile-Leu-Leu-Ala-Gln$^{20}$-Met-Gly-Arg-Ile-Ser$^{25}$-Leu-Phe-Ser-Cys-Leu$^{30}$-Lys-Asp-Arg-His-Asp$^{35}$-Phe-Gly-Phe-Pro-Gln$^{40}$-Glu-Glu-Phe-Asp-Gly$^{45}$-Asn, Y ist insbesondere Met$^{60}$-Ile-Gln-Gln-Ile-Phe$^{65}$-Asn-Leu-Phe-Ser-Thr$^{70}$-Lys-Asp-Ser-Ser-Ala$^{75}$-Ala-Trp-Asn-Glu-Ser$^{80}$-Leu-Leu-Asp-Lys-Phe$^{85}$ und Z ist insbesondere Met$^{112}$-Asn-Val-Glu$^{115}$-Ser-Ile-Leu-Ala-Val$^{120}$-Ser-Lys-Tyr-Phe-Gln$^{125}$-Arg-Ile-Thr-Leu-Tyr$^{130}$-Leu-Ser-Glu-Lys-Lys$^{135}$-Tyr-Ser-Pro-Cys-Ala$^{140}$-Trp-Glu-Ile-Val-Arg$^{145}$-Ala-Glu-Ile-Met-Arg$^{150}$-Ser-Leu-Thr-Leu-Leu$^{155}$-Thr-Asn-Leu-Gln-Glu$^{160}$-Arg-Leu-Arg-Asn-Lys$^{165}$-Asp.

Besonders bevorzugt ist das Hybrid-α-Interferon der Formel Met-Cys$^{1}$-Asp-Leu-Pro-Gln$^{5}$-Thr-His-Ser-Leu-Gly$^{10}$-Asn-Arg-Arg-Ala-Leu$^{15}$-Ile-Leu-Leu-Ala-Gln$^{20}$-Met-Gly-Arg-Ile-Ser$^{25}$-Leu-Phe-Ser-Cys-Leu$^{30}$-Lys-Asp-Arg-His-Asp$^{35}$-Phe-Gly-Phe-Pro-Gln$^{40}$-Glu-Glu-Phe-Asp-Gly$^{45}$-Asn-Gln-Phe-Gln-Lys$^{50}$-Ala-Gln-Ala-Ile-Ser$^{55}$-Val-Leu-His-Glu-Met$^{60}$-Ile-Gln-Gln-Ile-Phe$^{65}$-Asn-Leu-Phe-Ser-Thr$^{70}$-Lys-Asp-Ser-Ser-Ala$^{75}$-Ala-Trp-Asn-Glu-Ser$^{80}$-Leu-Leu-Asp-Lys-Phe$^{85}$-Ser-Thr-Glu-Leu-Tyr$^{90}$-Gln-Gln-Leu-Asn-Asp$^{95}$-Leu-Glu-Ala-Cys-Val$^{100}$-Ile-Gln-Glu-Val-Gly$^{105}$-Val-Glu-Glu-Thr-Pro$^{110}$-Leu-Met-Asn-Val-Glu$^{115}$-Ser-Ile-Leu-Ala-Val$^{120}$-Ser-Lys-Tyr-Phe-Gln$^{125}$-Arg-Ile-Thr-Leu-Tyr$^{130}$-Leu-Ser-Glu-Lys-Lys$^{135}$-Tyr-Ser-Pro-Cys-Ala$^{140}$-Trp-Glu-Ile-Val-Arg$^{145}$-Ala-Glu-Ile-Met-Arg$^{150}$-Ser-Leu-Thr-Leu-Leu$^{155}$-Thr-Asn-Leu-Gln-Glu$^{160}$-Arg-Leu-Arg-Asn-Lys$^{165}$-Asp.

<u>Beispiel 1</u>

60 bis 70 kg schwere Kälber, die an Durchfällen, verursacht durch Rotaviren, erkrankt waren, erhielten 3 Tage

nacheinander jeweils eine magensaftresistente Gelatinekapsel mit 5 mg eines Human-Hybrid-α-Interferons (= 0,1 bis 0,2 x $10^8$ IFE/mg). Die Durchfälle hörten nach zwei Tagen auf; in den Exkrementen konnten keine Viren mehr nachgewiesen werden. Unbehandelte Kontrolltiere starben.

Beispiel 2

Ein geeignetes Puffersystem zur erfindungsgemäßen Verabreichung des α-Interferons hat die folgende Zusammensetzung:

| | |
|---|---|
| 0,005 g | Human-Hybrid-α-Interferon (0,1-0,2·$10^8$IFE/mg) |
| 7,00 g | NaCl |
| 5,00 g | $NaHCO_3$ |
| 3,00 g | KCl |
| 40,00 g | Dextrose-Anhydrid |

Diese Mischung wird in 2 l Wasser gelöst und die Lösung gemäß Beispiel 1 Kälbern verabreicht. Das Ergebnis entspricht dem von Beispiel 1.

PATENTANSPRÜCHE

1. Verwendung von α-Interferonen zur Herstellung eines Arzneimittels für die Behandlung von Durchfällen.

2. Verwendung von α-Interferonen zur Herstellung eines Arzneimittels für die Behandlung von virusbedingten Durchfällen.

3. Magensaftresistente Arzneimittelzubereitungen, enthaltend ein α-Interferon.

4. Magensaftresistente Kapsel, gekennzeichnet durch einen Gehalt an einem α-Interferon.

5. Arzneimittel nach Anspruch 3 oder 4, enthaltend $10^5$ bis $10^8$ IFE eines α-Interferons.

6. Arzneimittel nach Anspruch 3 bis 5, dadurch gekennzeichnet, daß das α-Interferon ein Human-Hybrid-Interferon ist.

7. Arzneimittel nach Anspruch 3 bis 6, dadurch gekennzeichnet, daß das α-Interferon die Formel Met-Cys$^{1}$-Asp-Leu-Pro-Gln$^{5}$-Thr-His-Ser-Leu-Gly$^{10}$-Asn-Arg-Arg-Ala-Leu$^{15}$-Ile-Leu-Leu-Ala-Gln$^{20}$-Met-Gly-Arg-Ile-Ser$^{25}$-Leu-Phe-Ser-Cys-Leu$^{30}$-Lys-Asp-Arg-His-Asp$^{35}$-Phe-Gly-Phe-Pro-Gln$^{40}$-Glu-Glu-Phe-Asp-Gly$^{45}$-Asn-Gln-Phe-Gln-Lys$^{50}$-Ala-Gln-Ala-Ile-Ser$^{55}$-Val-Leu-His-Glu-Met$^{60}$-Ile-Gln-Gln-Ile-Phe$^{65}$-Asn-Leu-Phe-Ser-Thr$^{70}$-Lys-Asp-Ser-Ser-Ala$^{75}$-Ala-Trp-Asn-Glu-Ser$^{80}$-Leu-Leu-Asp-Lys-Phe$^{85}$-Ser-Thr-Glu-Leu-Tyr$^{90}$-Gln-Gln-Leu-Asn-Asp$^{95}$-Leu-Glu-Ala-Cys-Val$^{100}$-Ile-Gln-Glu-Val-Gly$^{105}$-Val-Glu-Glu-Thr-Pro$^{110}$-Leu-Met-Asn-Val-Glu$^{115}$-Ser-Ile-Leu-Ala-Val$^{120}$-Ser-Lys-Tyr-Phe-Gln$^{125}$-Arg-Ile-Thr-Leu-Tyr$^{130}$-Leu-Ser-Glu-Lys-Lys$^{135}$-Tyr-Ser-Pro-Cys-Ala$^{140}$-Trp-Glu-Ile-Val-Arg$^{145}$-Ala-Glu-Ile-Met-Arg$^{150}$-Ser-Leu-Thr-Leu-Leu$^{155}$-Thr-Asn-Leu-Gln-Glu$^{160}$-Arg-Leu-Arg-Asn-Lys$^{165}$-Asp hat.

Patentansprüche Österreich:

1. Verwendung von α-Interferonen zur Herstellung eines Arzneimittels für die Behandlung von Durchfällen.

2. Verwendung von α-Interferonen zur Herstellung eines Arzneimittels für die Behandlung von virusbedingten Durchfällen.